# EUROPEAN PATENT APPLICATION

(11) **EP 4 316 339 A1**
(43) Date of publication of application: **07.02.2024**
(21) Application number: 22780214.7
(22) Date of filing: 17.03.2022
(51) Int. Cl.: A61B 1/00, A61B 1/07, A61B 18/22

(54) **IRRADIATION PROBE SYSTEM AND IRRADIATION PROBE**

(30) Priority: 30.03.2021 JP 2021056454
(71) Applicant: FURUKAWA ELECTRIC CO., LTD., Tokyo 100-8322 (JP)
(72) Inventor: IWAMA, Masaki, Tokyo 100-8322 (JP); NOMURA, Yutaka, Tokyo 100-8322 (JP); MATSUSHITA, Shunichi, Tokyo 100-8322 (JP)
(74) Representative: SSM Sandmair
(86) International application number: PCT/JP2022/012466
(87) International publication number: WO 2022/209995

(57) **Abstract**

An irradiation probe system is, for example, an irradiation probe system including: an irradiation probe having a core and first cladding surrounding the core; at least one light source; and a coupling portion that couples light output by the at least one light source, to at least one of the core and the first cladding; the core includes a first input end portion at one end of an axis of the core, and an output end portion at the other end of the axis; and the first cladding includes a second input end portion at one end of the axis, and a leakage portion that is provided at a position separate from the second input end portion and leaks, radially outward, light transmitted inside the first cladding, from an outer peripheral surface of the first cladding.

## Description

### Field

The present invention relates to irradiation probe systems and irradiation probes.

### Background

A conventionally known probe for medical use emits laser light from a distal end of the probe (for example, Patent Literature 1).

### Citation List

### Patent Literature

Patent Literature 1: Japanese Unexamined Patent Application, Publication No. 2011-104199

### Summary

### Technical Problem

The position from which the light is emitted in the probe of Patent Literature 1 is just the distal end of the probe and the range irradiated with the light is thus limited to just a region near the distal end.

Enabling increase in the irradiation range of this type of irradiation probe would be beneficial. A cylindrical diffuser in Patent Literature 2 has been known as an irradiation probe with an increased irradiation range. The cylindrical diffuser, which enables uniform irradiation over a wide range in a longitudinal direction is suitable for treatment of a lesion generated in a fistula, such as an esophagus, or a bladder, such as a stomach.

Plural diffusers may be used for irradiation of an affected part with light in photodynamic therapy (PDT). For example, in a case that may be considered, a central part of a tumor is irradiated with light high in energy density from a fiber edge and a peripheral part of the tumor is irradiated over a wide range with light low in energy density by a cylindrical diffuser. In this case, more than one medical doctor holds more than one diffuser for treatment, raising the difficulty and cost of the surgery. As to the case, if a diffuser that enables irradiation of more than one part on its own were available, the difficulty and cost of the surgery would be decreased and burdens on both the medical doctor and the patient would thus be able to be reduced.

One of objects of the present invention is, for example, to obtain an irradiation probe system and an irradiation probe that have been improved, are novel, and enable irradiation of more than one location using just one irradiation probe.

### Solution to Problem

An irradiation probe system according to the present invention, for example, includes: an irradiation probe having a core and first cladding surrounding the core; at least one light source; and a coupling portion that couples light output by the at least one light source, to at least one of the core and the first cladding, wherein the core includes a first input end portion at one end of an axis of the core, and an output end portion that is at the other end of the axis, and the first cladding includes: a second input end portion at one end of the axis; and a leakage portion that is provided at a position separate from the second input end portion and leaks, radially outward, light transmitted inside the first cladding, from an outer peripheral surface of the first cladding.

In the irradiation probe system, light transmitted inside the core may be output from the output end portion.

The irradiation probe system may include, as the at least one light source: a light source that outputs light to be coupled to the core; and a light source that outputs light to be coupled to the first cladding.

In the irradiation probe system, the irradiation probe may include a first covering layer between the second input end portion and the leakage portion, the first covering layer surrounding the outer peripheral surface of the first cladding.

In the irradiation probe system, the first covering layer may include second cladding having a refractive index lower than that of the first cladding.

In the irradiation probe system, the irradiation probe may include an outer cover surrounding a surface of the irradiation probe, the surface being radially outermost.

In the irradiation probe system, the outer cover may be made of a resin material.

In the irradiation probe system, the irradiation probe may include a section at a position separate, along the axis, from the leakage portion, the section being where the outer peripheral surface of the first cladding is exposed.

In the irradiation probe system, the irradiation probe may include a second covering layer that surrounds the core or the first cladding, the second covering layer being on an opposite side of the leakage portion, the opposite side being opposite to a side where the first input end portion is along the axis, and that reduces coupling of light from outside to the core.

In the irradiation probe system, the second covering layer reduces output, along the axis, of the light transmitted inside the first cladding.

In the irradiation probe system, the irradiation probe may include a second covering layer that surrounds the core or the first cladding, the second covering layer being on an opposite side of the leakage portion, the opposite side being opposite to a side where the first input end portion is along the axis, and that reduces output, along the axis, of the light transmitted inside the first cladding.

The irradiation probe system may include a first optical element that directs, at the output end portion, light transmitted in the core, radially outward.

In the irradiation probe system, the first optical element may be configured to be detachable.

In the irradiation probe system, the leakage portion may include a recessed portion or a protruding portion provided on the outer peripheral surface of the first cladding.

In the irradiation probe system, the leakage portion may include particles or holes provided inside the first cladding.

In the irradiation probe system, the leakage portion may include a section where the outer peripheral surface of the first cladding changes in shape along the axis.

In the irradiation probe system, the leakage portion may include a curved portion of the first cladding.

In the irradiation probe system, the leakage portion may include a scattering layer that: is provided radially outside the first cladding; and propagates and scatters, radially outward, the light from the first cladding.

In the irradiation probe system, the first input end portion and the second input end portion may be arranged in a radial direction at one end of the axis, and the coupling portion may couple light output from one of the at least one light source to the core and couple light output from another one of the at least one light source to the first cladding.

In the irradiation probe system, the coupling portion may be optically connected to the core and the first cladding.

In the irradiation probe system, the coupling portion may include a fiber bundle that is a bundle of: a first transmission optical fiber that transmits light output by one of the at least one light source and coupled to the core; and a second transmission optical fiber that transmits light output by another one of the at least one light source and coupled to the first cladding.

In the irradiation probe system, the fiber bundle may be a tapered fiber bundle that is tapered toward the irradiation probe.

The irradiation probe system may include: a switchover mechanism that performs selective coupling of light output by the light source to the core or to the first cladding; and a first control unit that controls the selective coupling by the switchover mechanism.

The irradiation probe system may include: an operation input unit for a user, and the first control unit may control the selective coupling by the switchover mechanism according to input of an operation by the user to the operation input unit.

The irradiation probe system may include: plural light sources as the at least one light source, and a second control unit that performs switchover between light sources to output light, among the plural light sources.

In the irradiation probe system, the plural light sources may include a light source that outputs light to be coupled to the core and a light source that outputs light to be coupled to the first cladding.

In the irradiation probe system, the plural light sources may include plural light sources that output light to be coupled to the core or plural light sources that output light to be coupled to the first cladding.

The irradiation probe system may include: an operation input unit for a user, and the second control unit may perform switchover between light sources to output light, among the plural light sources, according to input of an operation by the user to the operation input unit.

The irradiation probe system may include a detection unit that detects light coupled to the output end portion, transmitted inside the core, and output from the first input end portion.

In the irradiation probe system, the irradiation probe may include a second optical element that couples light coming from outside, to the output end portion.

In the irradiation probe system, the second optical element may be configured to be detachable.

The irradiation probe system may include: a third optical element: having a reflection portion that reflects the second light output from the other end of the axis of the irradiation probe; and configured to be detachable from the irradiation probe, and the irradiation probe system may be configured so that the second light reflected by the reflection portion is coupled to the first cladding, at the other end.

In the irradiation probe system, the irradiation probe may include an outer cover that is removable or detachable.

In the irradiation probe system, the irradiation probe may include, as the outer cover, plural outer covers that are removable or detachable.

In the irradiation probe system, the irradiation probe may include, as the outer cover, plural outer covers that are at different positions along the axis.

In the irradiation probe system, the irradiation probe may include, as the outer cover, an outer cover covering the output end portion and an outer cover covering an outer periphery of the leakage portion.

In the irradiation probe system, the irradiation probe may include a fixed outer cover and an outer cover that covers the fixed outer cover removably or detachably.

In the irradiation probe system, the irradiation probe may include, as the outer cover, plural outer covers that have been layered over one another.

In the irradiation probe system, the plural outer covers that have been layered over one another may be configured to be removable one by one from outside.

In the irradiation probe system, the outer cover may be provided at a portion that comes into contact with a living body upon use for the living body.

In the irradiation probe system, the outer cover may be made of a biocompatible resin material.

An irradiation probe according to the present invention, for example, includes a core and first cladding surrounding the core, and the core includes: a first input end portion at one end of an axis of the core; and an output end portion that is at the other end of the axis, and the first cladding includes: a second input end portion at one end of the axis; and a leakage portion that is provided at a position separate from the second input end portion and leaks, radially outward, light transmitted inside the first cladding, from an outer peripheral surface of the first cladding.

In the irradiation probe, light transmitted inside the core may be output from the output end portion.

The irradiation probe may include a first covering layer between the second input end portion and the leakage portion, the first covering layer surrounding the outer peripheral surface of the first cladding.

In the irradiation probe, the first covering layer may include second cladding having a refractive index lower than that of the first cladding.

The irradiation probe may include an outer cover surrounding a surface of the irradiation probe, the surface being radially outermost.

In the irradiation probe, the outer cover may be made of a resin material.

In the irradiation probe, the leakage portion may include particles or holes provided inside the first cladding.

The irradiation probe may include an outer cover that may be removable or detachable.

### Advantageous Effects of Invention

The present invention enables, for example, obtainment of an irradiation probe system and an irradiation probe that have been improved and are novel.

### Brief Description of Drawings

FIG. 1 is a diagram of an exemplary schematic configuration of an irradiation probe system of an embodiment.
FIG. 2 is an exemplary and schematic sectional view (a partial side view) of a coupling portion and an irradiation probe, of the embodiment.
FIG. 3 is an exemplary and schematic sectional view of the coupling portion and part of the irradiation probe, of the embodiment.
FIG. 4 is an exemplary block diagram of the irradiation probe system of the embodiment.
FIG. 5 is a schematic sectional view of an example of first cladding of the irradiation probe of the embodiment.
FIG. 6 is a schematic sectional view of an example of the first cladding of the irradiation probe of the embodiment.
FIG. 7 is a schematic sectional view of an example of the first cladding of the irradiation probe of the embodiment.
FIG. 8 is a schematic side view of an example of a leakage portion of the irradiation probe of the embodiment.
FIG. 9 is a schematic sectional view of an example of the leakage portion of the irradiation probe of the embodiment.
FIG. 10 is a schematic sectional view of an example of a distal end portion of the irradiation probe of the embodiment.
FIG. 11 is a schematic sectional view of an example of the distal end portion of the irradiation probe of the embodiment.
FIG. 12 is a schematic sectional view of an example of the distal end portion of the irradiation probe of the embodiment.
FIG. 13 is a schematic sectional view of an example of the distal end portion of the irradiation probe of the embodiment.
FIG. 14 is a schematic sectional view of an example of the distal end portion of the irradiation probe of the embodiment.
FIG. 15 is an exemplary and schematic sectional view (a partial side view) of part of a transmission optical fiber, the coupling portion, and part of the irradiation probe, of the embodiment.
FIG. 16 is a schematic sectional view of an example of the distal end portion of the irradiation probe of the embodiment.
FIG. 17 is a schematic sectional view of an example of the distal end portion of the irradiation probe of the embodiment.
FIG. 18 is a schematic sectional view of an example of the distal end portion of the irradiation probe of the embodiment.
FIG. 19 is a schematic sectional view of an example of the irradiation probe of the embodiment.
FIG. 20 is an exemplary and schematic sectional view of the irradiation probe in FIG. 19 with its covering removed.
FIG. 21 is a schematic sectional view of an example of the irradiation probe of the embodiment.
FIG. 22 is a schematic sectional view of an example of the distal end portion of the irradiation probe of the embodiment.

### Description of Embodiments

An exemplary embodiment of the present invention will be disclosed hereinafter. Configurations of the embodiment and functions and results (effects) brought about by these configurations described hereinafter are just examples. The present invention may be implemented by configurations other than those disclosed hereinafter with respect to the embodiment. Furthermore, the present invention achieves at least one of various effects (including derivative effects) achieved by these configurations.

Examples described hereinafter include like components. Therefore, the configurations of these examples achieve like functions and effects based on the like components. Furthermore, the same reference sign will hereinafter be assigned to such like components and any redundant explanation thereof may be omitted.

Ordinals are assigned in this specification for convenience to distinguish between parts, portions, and functional units, for example, and do not indicate any order of priority or sequential order.

### Embodiment

### Configuration of Irradiation Probe System

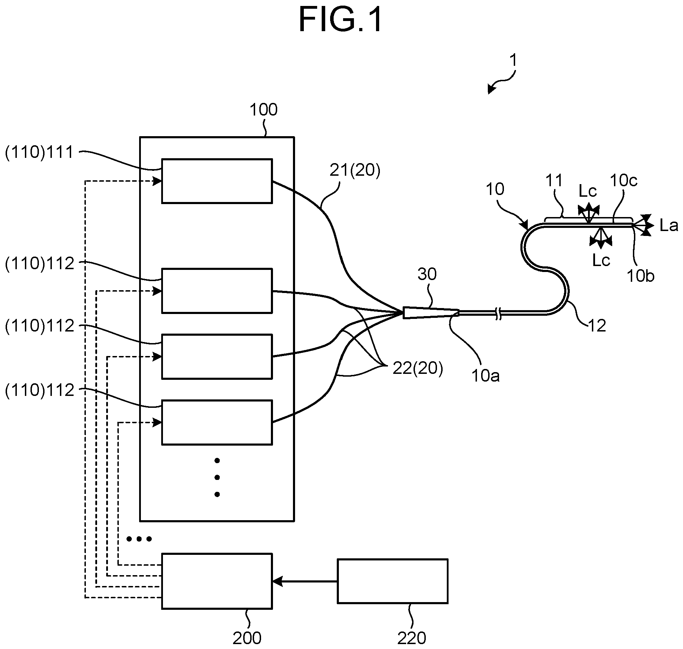

FIG. 1 is a schematic diagram of an irradiation probe system 1 of an embodiment. As illustrated in FIG. 1, the irradiation probe system 1 includes an optical output device 100, an irradiation probe 10, a control device 200, delivery optical fibers 20, a coupling portion 30, and an input unit 220.

The optical output device 100 has plural light source units 110. The light source units 110 each have a light source that outputs laser light, and an optical system that guides light from the light source to the delivery optical fiber 20 (both of which are not illustrated in the drawings). The light source includes, for example, a laser element that outputs laser light. Furthermore, the optical output device 100 has, for example, in this embodiment, the plural light source units 110, that is, light sources, but without being limited to this example, the optical output device 100 may just have at least one light source unit 110.

Each of the light source units 110 and the irradiation probe 10 are optically connected to each other via: the delivery optical fiber 20 provided correspondingly to that light source unit 110; and the coupling portion 30. That is, the delivery optical fibers 20 transmit light output by the light source units 110, to the coupling portion 30, and the coupling portion 30 couples the light transmitted by the delivery optical fibers 20, to the irradiation probe 10.

The irradiation probe 10 includes an optical fiber, has an elongated, cylindrical, and linear shape, and has flexibility. Furthermore, the irradiation probe 10 has an end portion 10a that is at one end of an axis of the irradiation probe 10, and an end portion 10b that is at the other end of the axis. The end portion 10a is an input end adjacent to the coupling portion 30, the input end being where light from the coupling portion 30 is input, and may also be referred to as a proximal end. Furthermore, the end portion 10b is positioned at an opposite end of the axis, the opposite end being opposite to the end where the end portion 10a is positioned, and may also be referred to as a distal end.

The irradiation probe 10 has a leakage portion 11 and a transmission portion 12. The leakage portion 11 is provided over a predetermined length along the axis, at a position separate from the end portion 10a, and is a section where light is leaked radially outward from an outer peripheral surface 10c of the irradiation probe 10. The transmission portion 12 is a section where light is transmitted: between the end portion 10a and the leakage portion 11; between the leakage portion 11 and the end portion 10b; or between two leakage portions 11 sandwiching an interval in a case where plural leakage portions 11 are provided with the interval therebetween along the axis. In this embodiment, for example, the leakage portion 11 is provided only in the section adjacent to the end portion 10b, but without being limited to this example, the leakage portion 11 may be provided separately from the end portion 10b.

The control device 200 is capable of controlling the light source units 110 to output light and stop outputting light, for example. Furthermore, the control device 200 is capable of controlling operation of a device or a part other than the light source units 110 in the irradiation probe system 1. The input unit 220 is included in a user interface operated by an operator (a user) and inputs instruction signals to the control device 200 according to input of operations by the operator. The input unit 220 is an example of an operation input unit.

### Configuration of Irradiation Probe

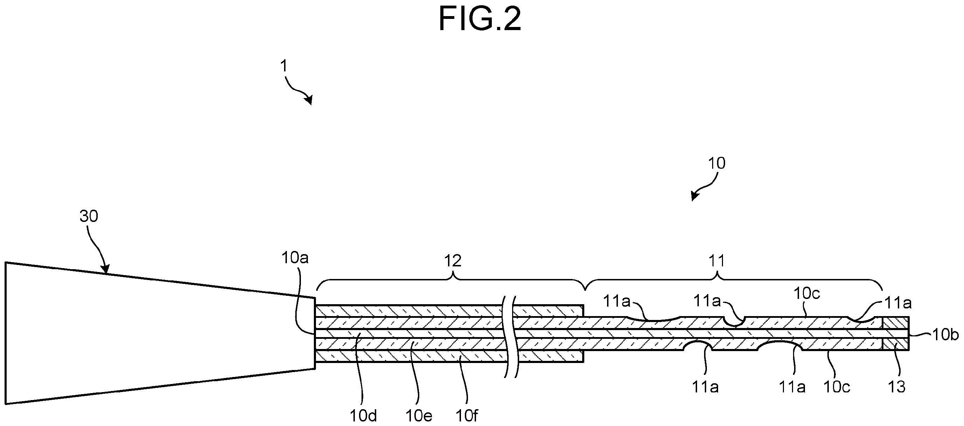

FIG. 2 is a diagram illustrating a cross section of the irradiation probe 10 and a side surface of the coupling portion 30. As mentioned above, the irradiation probe 10 has the transmission portion 12 and the leakage portion 11. In this embodiment, for example, the transmission portion 12 is configured as a double cladding fiber. That is, the transmission portion 12 has a core 10d extending along the axis, first cladding 10e surrounding the core 10d and extending along the axis, and second cladding 10f surrounding the first cladding and extending along the axis. The first cladding 10e has a refractive index lower than a refractive index of the core 10d and the second cladding 10f has a refractive index lower than the refractive index of the first cladding 10e.

Light input via the coupling portion 30 is transmitted inside each of the core 10d and the first cladding 10e of the transmission portion 12. Light transmitted inside the core 10d will hereinafter be referred to as first light and light transmitted inside the first cladding 10e will be referred to as second light.

The second cladding 10f may be, for example, an outer cover made of a resin material, such as a synthetic resin material having flexibility. Furthermore, the second cladding 10f may be air. In this case, the second cladding 10f does not exist between the end portion 10a and the leakage portion 11 and the outer peripheral surface 10c of the first cladding 10e is exposed at least from the second cladding 10f in the transmission portion 12. The transmission portion 12 may have an outer cover surrounding the second cladding 10f. In these configurations, the second cladding 10f or the outer cover is an example of a first covering layer surrounding the outer peripheral surface 10c of the first cladding 10e at least between the end portion 10a and the leakage portion 11. This first covering layer enables reduction of leakage of the second light from the first cladding 10e. Furthermore, a portion of the irradiation probe 10 may be wholly covered with an outer cover, the portion being a portion to be inserted into a body.

The leakage portion 11 leaks the second light transmitted inside the first cladding 10e, radially outward from the outer peripheral surface 10c of the first cladding 10e. In this embodiment, the leakage portion 11 does not have second cladding. Furthermore, in this embodiment, for example, recessed portions 11a are provided on the outer peripheral surface 10c of the first cladding 10e in the leakage portion 11. In this case, the second light is refracted at the recessed portions 11a and thus the traveling direction of the second light is changed, that is, the second light is scattered, and the second light leaks radially outward from the outer peripheral surface 10c. The outer peripheral surface 10c may be provided with protruding portions, instead of the recessed portions 11a. These protruding portions may be, for example, portions between the recessed portions 11a. In this embodiment, these recessed portions 11a or protruding portions promote the leakage of the second light radially outward from the first cladding 10e. Adjusting, as appropriate, specifications, such as the positions to provide the recessed portions 11a or protruding portions, the density at which the recessed portions 11a or protruding portions are provided, and/or the sizes or depths of the recessed portions 11a or protruding portions, enables appropriate adjustment of the distribution of levels of leakage of the second light along the axis of the leakage portion 11.

The first light transmitted through the core 10d, on the other hand, is output from the end portion 10b. The end portion 10b of the core 10d is an example of an output end portion. The first cladding 10e enables reduction of leakage of the first light from the outer peripheral surface of the core 10d.

Accordingly, the irradiation probe 10 is capable of outputting first light La (see FIG. 1) via the end portion 10b of the core 10d and is capable of outputting second light Lc (see FIG. 1) radially outward via the leakage portion 11. That is, the irradiation probe system 1 of the embodiment enables irradiation of a region facing the end portion 10b of the irradiation probe 10 with the first light, as well as irradiation of a region facing the outer peripheral surface 10c of the irradiation probe 10 with the second light. As described above, the embodiment enables increase in the range irradiated with light by the irradiation probe 10 as compared to a configuration in which the range irradiated with light is limited to a region near the end portion of the axis. The irradiation probe system 1 may emit the first light and the second light simultaneously or may emit the first light and the second light selectively.

Furthermore, as illustrated in FIG. 2, in this embodiment, the irradiation probe 10 has a covering layer 13 surrounding the core 10d, the covering layer 13 being between the first cladding 10e of the leakage portion 11 and the end portion 10b of the core 10d. This covering layer 13 surrounds the core 10d at the opposite end of the axis, the opposite end being on the opposite side of the leakage portion 11, the opposite side being opposite to the side where the end portion 10a is, and thereby reduces coupling of reflected light of the second light to the core 10d, for example. In this case, the covering layer 13 may have an absorption layer that absorbs light. The absorption layer may be configured as, for example, a black oxide finished layer made of copper oxide covering. This covering layer 13 enables reduction of weakening of output of the first light, the weakening being caused by interference between the first light and the second light input to the core 10d. The covering layer 13 may be configured to surround the outer periphery of the first cladding 10e. The covering layer 13 is an example of a second covering layer.

### Configuration of Coupling Portion

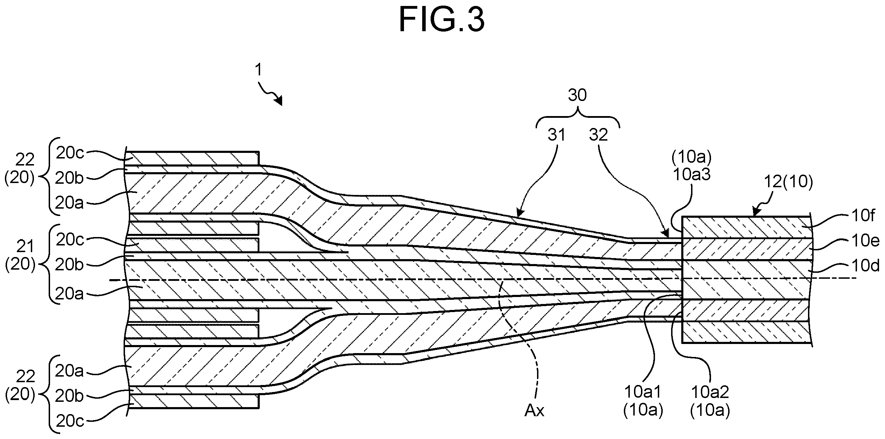

FIG. 3 is a sectional view of the coupling portion 30 and part of the irradiation probe 10. The coupling portion 30 is connected to the irradiation probe 10 by fusion or bonding, for example. That is, the coupling portion 30 is connected to the irradiation probe 10 optically and mechanically. An end surface of the coupling portion 30 and an end surface of the irradiation probe may be configured to contact each other by means of another member (not illustrated in the drawings).

Furthermore, as illustrated in FIG. 3, the coupling portion 30 has a fiber bundle that is a bundle of the plural delivery optical fibers 20. The fiber bundle is, for example, a tapered fiber bundle that is tapered toward the irradiation probe 10. In FIG. 3, for example, end portions of seven delivery optical fibers 20 having the same diameter are bundled together in a close-packed manner. The delivery optical fibers 20 each have a core 20a, cladding 20b surrounding the core 20a, and an outer cover 20c surrounding the cladding 20b. A portion having the outer covers 20c removed therefrom composes the coupling portion 30 having a tapered portion 31 and a straight lined portion 32. The tapered portion 31 gradually decreases in outer diameter toward the irradiation probe 10 and the cladding 20b and the cladding 20b of adjacent ones of the delivery optical fibers 20 in the tapered portion 31 are integrated with each other. The coupling portion 30 may have no straight lined portion 32. Furthermore, the number of the optical fibers bundled together is not limited to seven, and the fiber bundle may just have end portions of at least two delivery optical fibers 20 that have been bundled together. In addition, the fiber bundle may have optical fibers optically connected respectively to the delivery optical fibers 20 and different from the delivery optical fibers 20.

As illustrated in FIG. 3, an end surface 10a1 of the core 10d of the irradiation probe 10 faces an edge of a delivery optical fiber 21 positioned in the center of the bundle. As illustrated in FIG. 1, the delivery optical fiber 21 is optically connected to a light source unit 111 (110). The coupling portion 30 couples light output by the light source unit 111 and transmitted by the delivery optical fiber 21, to the core 10d. A light source of the light source unit 111 outputs the light coupled to the core 10d, that is, the first light. The end surface 10a1 is part of the end portion 10a and is an example of a first input end portion. The delivery optical fiber 21 is an example of a first transmission optical fiber.

Furthermore, as illustrated in FIG. 3, an end surface 10a2 of the first cladding 10e of the irradiation probe 10 faces an edge of a delivery optical fiber 22 shifted radially outward from an optical axis Ax. As illustrated in FIG. 1, the delivery optical fiber 22 is optically connected to a light source unit 112 (110). The coupling portion 30 couples light output by the light source unit 112 and transmitted by the delivery optical fiber 22, to the first cladding 10e. A light source of the light source unit 112 outputs the light coupled to the first cladding 10e, that is, the second light. The end surface 10a2 is an example of a second input end portion that: is part of the end portion 10a; is arranged beside the end surface 10a1 in a radial direction at the end portion 10a; and positioned more radially outward than the end surface 10a1. The delivery optical fiber 22 is an example of a second transmission optical fiber.

Furthermore, as illustrated in FIG. 3, in this embodiment, for example, the first cladding 10e has a plurality of the delivery optical fibers 22 optically connected to the first cladding 10e. Therefore, in a case where a plurality of the light source units 112 concurrently output light, light output from the plurality of the light source units 112, that is, the second light is coupled to the first cladding 10e via the coupling portion 30. Although not illustrated in the drawings, a plurality of the light source units 111 may be optically connected to the core 10d via the coupling portion 30 and a plurality of the delivery optical fibers 21 and light output from the plurality of the light source units 111, that is, the first light may be coupled to the core 10d.

Furthermore, as illustrated in FIG. 3, no edges of the delivery optical fibers 20 face an end surface 10a3 of the second cladding 10f. The end surface 10a3 may be provided with a shield so that, for example, external light is not coupled to the second cladding 10f.

Furthermore, the numerical aperture of the light source unit 111 at an optical connection between the light source unit 111 and the delivery optical fiber 21 is set to be approximately the same as or slightly smaller than the numerical aperture of the delivery optical fiber 21. In addition, the numerical aperture of the delivery optical fiber 21 at an optical connection between the delivery optical fiber 21 (the coupling portion 30) and the end portion 10a of the irradiation probe 10 is set to be approximately the same as or slightly smaller than the numerical aperture of the core 10d. Similarly, the numerical aperture of the light source unit 112 is set to be approximately the same as or slightly smaller than the numerical aperture of the delivery optical fiber 22. Furthermore, the numerical aperture of the delivery optical fiber 22 at an optical connection between the delivery optical fiber 22 (the coupling portion 30) and the end portion 10a of the irradiation probe 10 is set to be approximately the same as or slightly smaller than the numerical aperture of the first cladding 10e. Setting the numerical apertures as described above reduces losses at the optical connections for the first light and second light. The numerical aperture of the first cladding 10e is larger than the numerical aperture of the core 10d. Therefore, the numerical aperture of the light source unit 112 is larger than the numerical aperture of the light source unit 111. The settings of the numerical apertures of the light source units 110 may be changed according to, for example, the design of the numerical aperture of a lens system of an output unit not illustrated in the drawings.

As described above, the control device 200 is capable of performing switchover between output of light and stoppage of output of light by the light source units 110. Therefore, the control of the operation of the light source units 110 by the control device 200 enables switchover between: a state where the first light output by the light source unit 111 is output from the end portion 10b of the irradiation probe 10; a state where the second light output by the light source unit 112 is output from the outer peripheral surface 10c of the leakage portion 11 of the irradiation probe 10; a state where the first light output by the light source unit 111 is output from the end portion 10b of the irradiation probe 10 and the second light output by the light source unit 112 is output from the outer peripheral surface 10c of the irradiation probe 10; and a state where none of the first light and second light is output from the irradiation probe 10.

Furthermore, by changing the number of the light source units 112 to output the second light, the control device 200 is able to change the intensity of the second light leaked from the outer peripheral surface 10c in the leakage portion 11. In addition, in a case where the irradiation probe system 1 includes a plurality of the light source units 111 that output the first light, by changing the number of the light source units 111 that output the first light, the control device 200 is able to change the intensity of the first light output from the end portion 10a.

Furthermore, the control device 200 is able to perform switchover between the above described operation states and to change the intensity, on the basis of an instruction signal based on input of an operation by an operator to the input unit 220.

### Control of Irradiation System

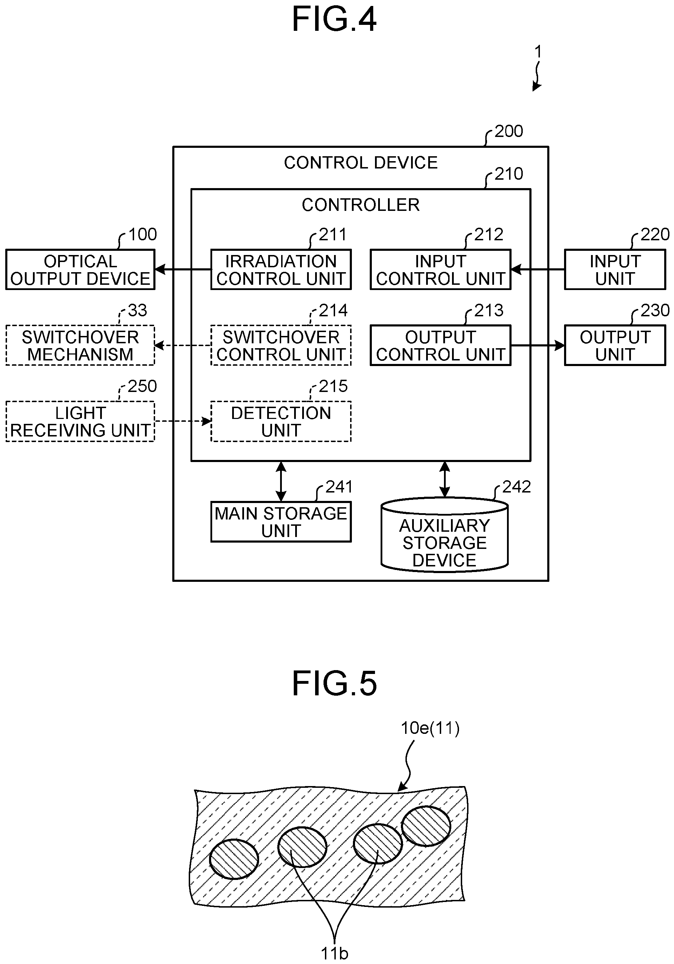

FIG. 4 is a block diagram of the irradiation probe system 1. As illustrated in FIG. 4, the irradiation probe system 1 includes the control device 200, the input unit 220, and an output unit 230. The input unit 220 and the output unit 230 construct a user interface for a user or an operator. The input unit 220 is, for example, an operation unit, such as a remote controller, a switch box, or a joystick, or an input device, such as a keyboard, a touch panel, a mouse, switches, or operation buttons. The output unit 230 is, for example, an output device using an image, printing, or sound, such as a display, a printer, a lamp, or a speaker.

Furthermore, the control device 200 has a controller 210, a main storage unit 241, and an auxiliary storage device 242.

The controller 210 is, for example, a processor (a circuit), such as a central processing unit (CPU). The main storage unit 241 is, for example, a random access memory (RAM) or a read only memory (ROM). Furthermore, the auxiliary storage device 242 is, for example, a nonvolatile rewritable storage device, such as a solid state drive (SSD) or a hard disk drive (HDD).

By reading programs stored in the main storage unit 241 or the auxiliary storage device 242 and executing their processes, the controller 210 operates as an irradiation control unit 211, an input control unit 212, and an output control unit 213. The programs may each be provided by being recorded in a computer-readable recording medium, as a file having an installable format or an executable format. The recording medium may also be referred to as a program product. The programs, values to be used in arithmetic processing by the processor, and information, such as maps and tables, may be stored in the main storage unit 241 or the auxiliary storage device 242 beforehand, or may be stored in a storage unit of a computer connected to a communication network and stored into the auxiliary storage device 242 by being downloaded via the communication network. The auxiliary storage device 242 stores data written into the auxiliary storage device 242 by the processor. Furthermore, at least part of arithmetic processing executed by the controller 210 may be executed by hardware. In this case, the controller 210 may include, for example, a field programmable gate array (FPGA) or an application specific integrated circuit (ASIC) .

The irradiation control unit 211 is capable of controlling output of light and stoppage of output of light individually for the respective light source units 110 included in the optical output device 100. The irradiation control unit 211 is able to performs switchover between light source units 110 to output light, among the plural light source units 110 (light sources) according to input of an operation by an operator to the input unit 220. The irradiation control unit 211 is an example of a second control unit.

The input control unit 212 receives an input signal from the input unit 220. Furthermore, the input control unit 212 may control the input unit 220 to enable input of a predetermined operation.

The output control unit 213 controls the output unit 230 to execute predetermined output.

A switchover control unit 214, a detection unit 215, a switchover mechanism 33, and a light receiving unit 250 will be described later.

Examples of Configuration of Leakage Portion

### (Modified Examples)

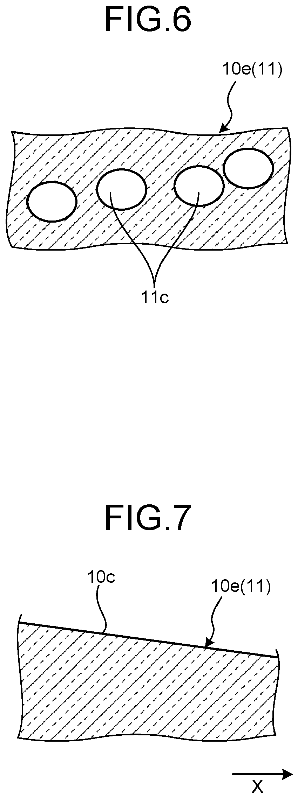

FIG. 5 and FIG. 6 are each a sectional view of an example of a configuration of the leakage portion 11. In the example of FIG. 5, particles 11b are included in the first cladding 10e of the leakage portion 11, and in the example of FIG. 6, holes 11c are included in the first cladding 10e of the leakage portion 11. The particles 11b and the holes 11c are, for example, nanostructures having a diameter of 100 nm or less. The particles 11b may be, for example, microparticles, or filler, such as microtubes. In any of these cases, the traveling direction of the second light is changed by the particles 11b or the holes 11c, that is, the second light is scattered, and it thus becomes easier for the second light to be leaked radially outward from the outer peripheral surface 10c.

FIG. 7 is a sectional view of another example of the configuration of the leakage portion 11. In the example of FIG. 7, the outer peripheral surface 10c is inclined with respect to an axial direction X of the irradiation probe 10. The outer peripheral surface 10c is, for example, a tapered surface. The second light thus becomes incident on an area where the shape of the outer peripheral surface 10c changes in the axial direction X by exceeding the critical angle, and it thus becomes easier for the second light to be leaked radially outward from the outer peripheral surface 10c.

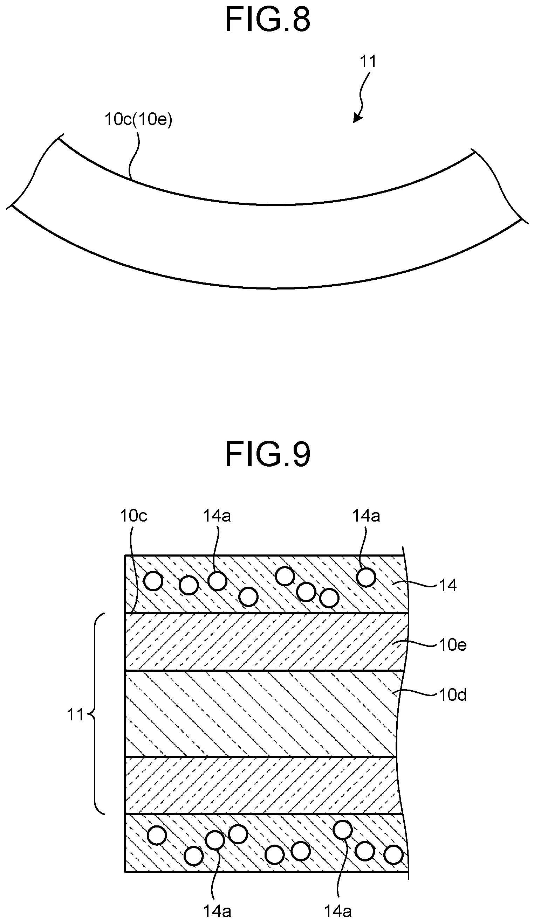

FIG. 8 is a sectional view of another example of the configuration of the leakage portion 11. In the example FIG. 8, the leakage portion 11 is curved. Light is more easily leaked from curved parts. That is, the configuration in FIG. 8 also facilitates leakage of the second light radially outward from the outer peripheral surface 10c.

FIG. 9 is a sectional view of another example of the configuration of the leakage portion 11. In the example of FIG. 9, the leakage portion 11 has a scattering layer 14 surrounding the first cladding 10e. The scattering layer 14 has a refractive index set to be approximately the same as or slightly higher than the refractive index of the first cladding 10e. Furthermore, the scattering layer 14 includes scattering elements 14a that are particles or holes. In this case, the second light that has reached the interface between the first cladding 10e and the scattering layer 14 enters the scattering layer 14 and leaks radially outward from the scattering elements 14a. This configuration has, for example, an advantage that the scattering layer 14 enables a place to be set or changed as appropriate, the place being a place where the second light leaks out from, a place where the second light easily leaks out from, or a place where the intensity of light leaked becomes high. Furthermore, in a case where the scattering layer 14 applies adequate pressure to the first cladding 10e radially inward, it becomes easier for the second light to be leaked from the pressured part.

Furthermore, the configurations illustrated in FIG. 5 to FIG. 9 may be implemented in any combination as appropriate in a single irradiation probe 10.

Examples of Configuration of Distal End of Core of Irradiation Probe (Modified Examples)

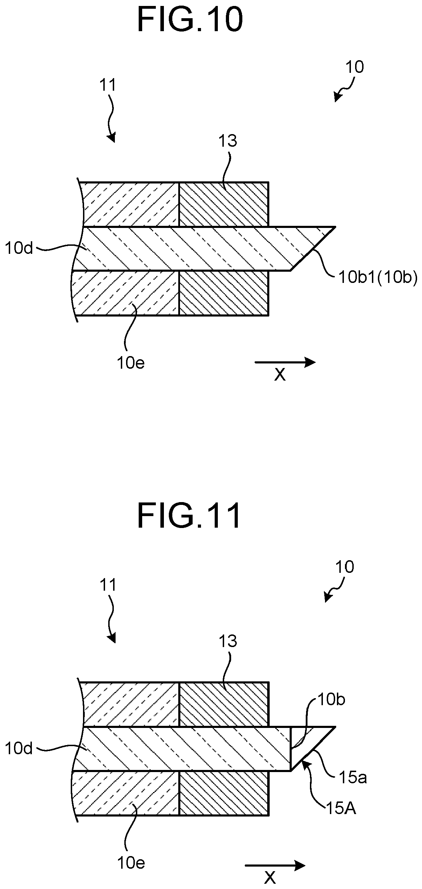

FIG. 10 to FIG. 14 are each a sectional view of a distal end portion of an irradiation probe 10 of a modified example of the embodiment.

In the modified example illustrated in FIG. 10, an end portion 10b of a core 10d has an inclined surface 10b1 provided thereon, the inclined surface 10b1 being inclined with respect to an axial direction X. In this case, first light is reflected radially outward at the inclined surface 10b1. The direction in which the first light is reflected is able to be adjusted as appropriate by the inclination of the inclined surface 10b1. This configuration is effective in a case where a region to be irradiated with the first light is desired to be set in the radial direction of the irradiation probe 10. The inclined surface 10b1 is an example of a first optical element.

In the modified example illustrated in FIG. 11, an end portion 10b of a core 10d has an optical element 15A attached to the end portion 10b by, for example, fusion or bonding, the optical element 15A having an inclined surface 15a similar to the inclined surface 10b1 illustrated in FIG. 10. In this case, the optical element 15A has a refractive index that is set to be approximately the same as or higher than a refractive index of the core 10d. This configuration also has effects similar to those of the configuration in FIG. 10. Furthermore, the configuration in FIG. 11 has an advantage that the optical element 15A to illuminate an appropriate region is able to be retrofitted to the core 10d of the irradiation probe 10. The optical element 15A and the inclined surface 15a are an example of the first optical element.

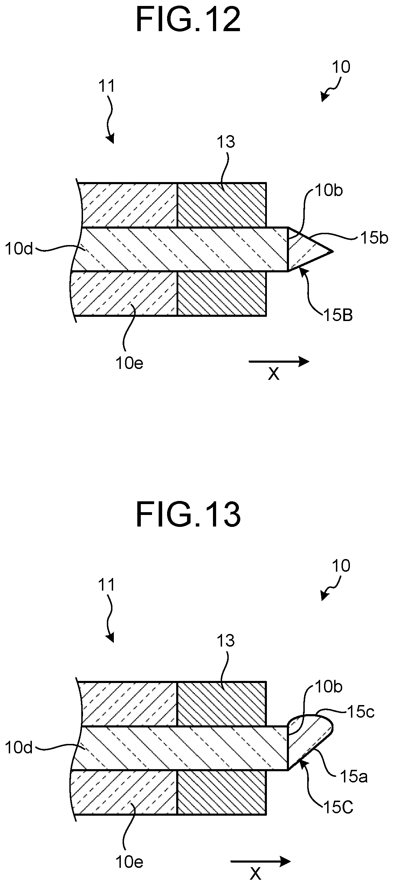

In the modified example illustrated in FIG. 12, an end portion 10b of a core 10d has an optical element 15B attached to the end portion 10b by, for example, fusion or bonding, the optical element 15B having a conical surface 15b pointed in an axial direction X. In this case, the optical element 15A has a refractive index that is set to be approximately the same as or higher than a refractive index of the core 10d. This configuration also has effects similar to those of the configurations in FIG. 10 and FIG. 11. Furthermore, the configuration in FIG. 12 has an advantage that a wider range around the end portion 10a, for example, over its entire circumference, is able to be irradiated with first light. The optical element 15B and the conical surface 15b are an example of the first optical element.

In the modified example illustrated in FIG. 13, an end portion 10b of a core 10d has an optical element 15C attached to the end portion 10b by, for example, fusion or bonding, the optical element 15C having, in addition to an inclined surface 15a similar to the one in FIG. 11, a lens 15c that refracts light reflected at the inclined surface 15a. In this case, there is an advantage that setting specifications of the lens 15c enables, for example, appropriate adjustment of the irradiation state of first light, such as narrowing of the range irradiated with the first light, widening of the irradiation range, appropriate adjustment of the irradiated position, increase in the irradiation intensity, or decrease in the irradiation intensity. The optical element 15C and the lens 15c are an example of the first optical element.

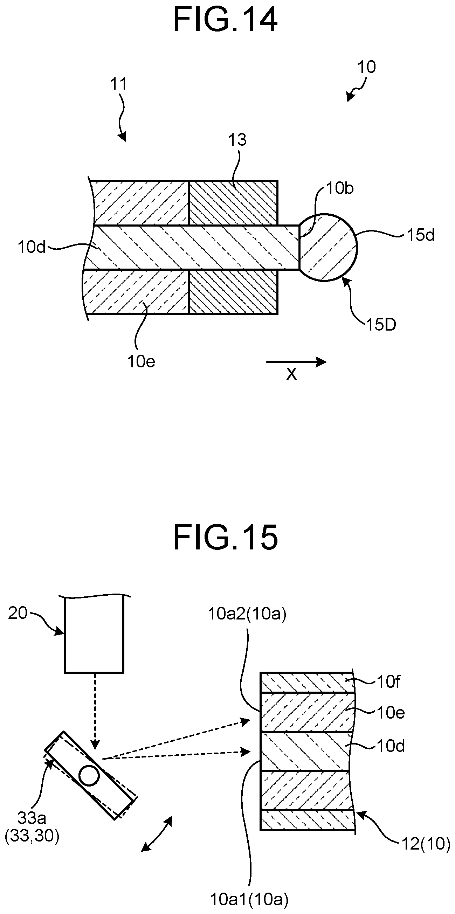

In the modified example illustrated in FIG. 14, an end portion 10b of a core 10d has an optical element 15D attached to the end portion 10b by, for example, fusion or bonding, the optical element 15D having a convex curved surface 15d like a spherical surface. In this case, similarly to the lens 15c in FIG. 3, there is an advantage that the irradiation state of first light is able to be adjusted as appropriate according to the shape of the convex curved surface 15d. The optical element 15D and the convex curved surface 15d are an example of the first optical element.

### Detection of Returned Light from Core of Irradiation Probe

The irradiation probe system 1 may also include the light receiving unit 250 as illustrated in FIG. 4. The light receiving unit 250 is optically connected to the end portion 10a of the core 10d, the end portion 10a being at a proximal end of the core 10d, via an optical coupler not illustrated in the drawings and the delivery optical fiber 21, and the light receiving unit 250 is capable of receiving foreign light that has been coupled to the end portion 10b of the core 10d, the end portion 10b being at a distal end of the core 10d, transmitted in the core 10d, and output from the end portion 10a. The light receiving unit 250 is, for example, a light receiving element. Furthermore, the controller 210 is capable of operating as the detection unit 215 according to a program and detecting the intensity of the received foreign light. In this case, in combination with, for example, the inclined surface 10b1 or the optical element 15A, 15B, 15C, or 15D illustrated in FIG. 10 to FIG. 14, foreign light coming from a specific range or direction is able to be received by the light receiving unit 250 and the intensity of the foreign light is able to be detected by the detection unit 215. In this case, the inclined surface 10b1, the inclined surface 15a, the conical surface 15b, the lens 15c, the convex curved surface 15d, and the optical elements 15A to 15D are examples of a second optical element that couples light coming from outside, to the end portion 10b.

### Another Configuration of Coupling Portion (Modified Example)

FIG. 15 is a sectional view (a partial side view) of part of a delivery optical fiber 20, a coupling portion 30, and part of an irradiation probe 10. In an example of FIG. 15, the coupling portion 30 has a switchover mechanism 33 including a mirror 33a that is able to be changed in orientation. The switchover mechanism 33 is configured to be able to change the angle of light received from the delivery optical fiber 20 between an angle at which the light is coupled to a core 10d of the irradiation probe 10 and an angle at which the light is coupled to first cladding 10e of the irradiation probe 10. The switchover mechanism 33 has an actuator (not illustrated in the drawings) that is capable of electrically changing the angle of the mirror 33a. In this case, the controller 210 (see FIG. 4) of the control device 200 operates as the switchover control unit 214 according to a program and controls the actuator of the switchover mechanism 33 to thereby change the angle of the mirror 33a and to enable switchover between a state where the light from the delivery optical fiber 20 is coupled to the core 10d and a state where the light from the delivery optical fiber 20 is coupled to the first cladding 10e. In other words, the switchover mechanism 33 is capable of selectively coupling light output by a light source unit 110 to the core 10d or the first cladding 10e. The switchover control unit 214 is capable of controlling this selective coupling by the switchover mechanism 33. Furthermore, the switchover control unit 214 is capable of controlling the selective coupling by the switchover mechanism 33 according to input of an operation by an operator to the input unit 220. This configuration enables switchover between use of the light source unit 110 as a light source of first light and use of the light source unit 110 as a light source of second light. The switchover control unit 214 is an example of a first control unit.

### First Modified Example of Optical Element that is Detachable

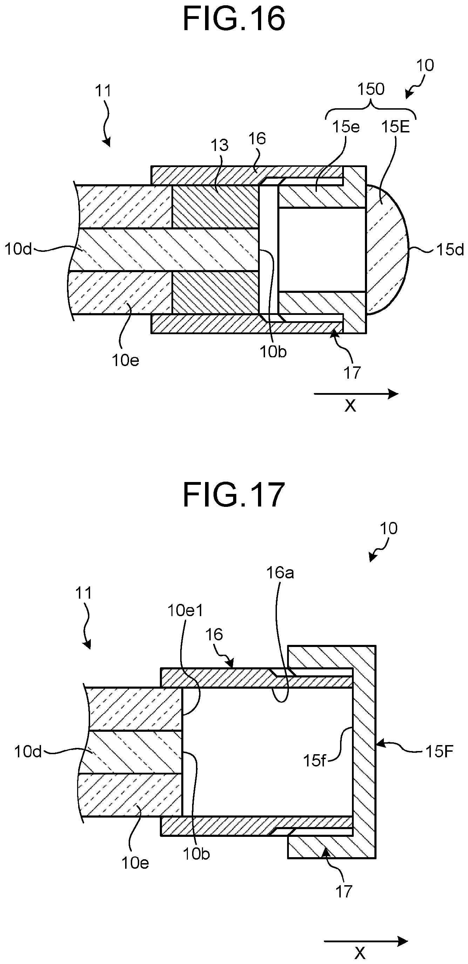

FIG. 16 is a sectional view of a distal end portion of an irradiation probe 10 of a modified example of the embodiment. In the example of FIG. 16, an optical element 15E that functions as the first optical element or second optical element is detachably provided at the distal end portion of the irradiation probe 10. For example, a socket 16 that protrudes from an end portion 10b and is cylindrical is provided at the distal end portion of the irradiation probe 10. Furthermore, the optical element 15E and a cylinder 15e configured to be detachable from the socket 16 are included in an attachment 150. The socket 16 and the cylinder 15e, that is, the attachment 150, are configured to be detachable from each other via a screw mechanism 17. The screw mechanism 17 may also be referred to as an attachment and detachment mechanism. Furthermore, the socket 16 may also be referred to as an adapter. In a state where the attachment 150 has been removed, first light transmitted through the core 10d is output in an axial direction from the end portion 10b. That is, this configuration enables switchover between a state where the attachment 150 has been removed and the first light is output in the axial direction from the end portion 10b and a state where the attachment 150 has been attached and the first light is output from the end portion 10b via the optical element 15E or foreign light is coupled to the core 10d via the optical element 15E. The attachment and detachment mechanism that enables the optical element 15E to be detachable is not limited to the screw mechanism, and may be another mechanism, such as a snap-fit mechanism. Furthermore, in the example of FIG. 16, the optical element 15E has a convex curved surface 15d, but the optical element 15E may have another surface or shape, such as, for example, an inclined surface or a conical surface.

### Second Modified Example of Optical Element that is Detachable

FIG. 17 is a sectional view of a distal end portion of an irradiation probe 10 of a modified example of the embodiment. In the example of FIG. 17, an optical element 15F is detachably provided at a distal end of the irradiation probe 10. For example, the optical element 15F is configured to be detachable from a socket 16 via a screw mechanism 17 serving as the attachment and detachment mechanism, the socket 16 protruding from an end portion 10b and being cylindrical. The optical element 15F is used in a case where first light is not emitted from the end portion 10b in an axial direction. An inner end surface 15f of the optical element 15F, the inner end surface 15f facing the end portion 10b, and an inner peripheral surface 16a of the socket 16 are configured to reflect second light. Therefore, the second light emitted from an end portion 10e1 or the end portion 10b reaches the inner end surface 15f directly or via the inner peripheral surface 16a, is reflected at the inner end surface 15f, and is coupled to first cladding 10e again directly or via the inner peripheral surface 16a. The second light coupled to the first cladding 10e travels back toward the end portion 10a through the first cladding 10e and leaks from an outer peripheral surface 10c (see FIG. 1 and FIG. 2) of the first cladding 10e in a leakage portion 11. This configuration has an effect of, for example, enabling increase in output efficiency of the second light from the leakage portion 11 and reduction in energy loss, and reduction of variation in irradiation intensity of the second light depending on the location in the leakage portion 11, as compared to a case where the first light is not emitted in the axial direction and the optical element 15F is not attached. The optical element 15F is an example of a third optical element. Furthermore, the inner end surface 15f is an example of a reflection portion and may also be referred to as a reflection surface.

### First Modified Example of Outer Cover that is Removable

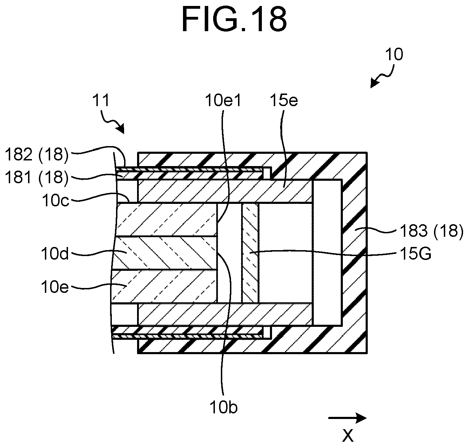

FIG. 18 is a sectional view of a distal end portion of an irradiation probe 10 of a modified example of the embodiment. In the example of FIG. 18, the irradiation probe 10 has plural outer covers 18 positioned on the outer periphery or at a distal end of the irradiation probe 10. In this modified example, the irradiation probe 10 includes three outer covers 181, 182, and 183, as the outer covers 18, but the number of the outer covers 18 is not limited to three and may be two or less, or four or more.

The outer covers 18 protect the irradiation probe 10. The outer covers 18 transmit light from portions respectively covered with the outer covers 18, that is, light from the outer periphery of the leakage portion 11 or from an end portion 10b. The outer covers 18 are made of, for example, a synthetic resin material or synthetic resin materials having biocompatibility. Furthermore, the outer covers 18 may differ in hardness (softness) depending on locations of the outer covers 18. For example, part of the outer covers 18, the part covering the distal end of the irradiation probe 10, may be made of a comparatively hard material, and part of the outer covers 18, the part covering the outer periphery of the irradiation probe 10, may be made of a comparatively soft material.

Furthermore, the outer covers 18 are provided to be at least partially removable or detachable. This configuration enables removal or replacement of part or all of the outer covers 18 of the irradiation probe 10, for example, in a case where the outer covers 18 become unclean by use for a living body or the outer covers 18 are damaged by hitting an object, the part being part that has become unclean or part that has been damaged, and the configuration thus enables the irradiation probe 10 to be maintained hygienic comparatively easily without cleaning or sterilization of the whole irradiation probe 10. In this specification, a structure in which the outer covers 18 are removable means a structure enabling a state to be restored, the state being similar to a state before removal of the outer covers 18, by attachment of the outer covers 18 that have been removed or attachment of outer covers 18 different from the outer covers 18 that have been removed, to portions from which the outer covers 18 have been removed. Furthermore, a structure in which the outer covers 18 are removable means a structure enabling the outer covers 18 to be removed without a problem of, for example, damaging portions from which the outer covers 18 are removed.

In the example of FIG. 18, the outer cover 181 has a tubular shape and covers the outer periphery of part of the irradiation probe 10, the part excluding the distal end of the irradiation probe 10. The outer cover 181 is, for example, unremovably fixed to the irradiation probe 10 by bonding. A distal end portion of the outer cover 181 is attached to the outer periphery of a cylinder 15e that holds an optical element 15G. The optical element 15G is, for example, a window or a mirror. The cylinder 15e may also be referred to as a holding member for the outer covers 18. The configuration of this distal end portion is not limited to this modified example, and the distal end portion may be configured like in the embodiment or any of the modified examples described above.

The outer cover 182 covers the periphery of the outer cover 181. That is, in this modified example, the outer cover 181 and the outer cover 182 are layered over each other in a radial direction. The outer cover 182 is, for example, a heat-shrinkable tube made of a synthetic resin material. In this case, the irradiation probe 10 is relatively inserted in an axial direction into the outer cover 182 having an inner diameter larger than an outer diameter of the outer cover 181 in an initial state before heating, the outer cover 182 being tubular, and the outer cover 182 thereafter shrinks thermally by the heating and covers the outer cover 181. The outer cover 182 elastically presses the outer cover 181 radially inward by shrinkage upon the heating and the outer cover 182 is fixed to the periphery of the outer cover 181 by frictional force associated with the pressing force. Furthermore, a user is able to remove the outer cover 182 from the outer cover 181 by, for example, making a cut in a portion of the outer cover 182 and tearing apart the outer cover 182 from the cut. That is, the outer cover 182 is removably provided. Furthermore, after the outer cover 182 has been removed, another outer cover 182 may be attached to cover the outer cover 181 by the same procedure described above, that is, by insertion and thermal shrinkage. That is, the outer covers 18 include the outer cover 182 that is both removable and detachable. The outer cover 181 inside the outer cover 182 may be removable or detachable from the irradiation probe 10.

Furthermore, the outer cover 183 is fitted to cover the distal end of the irradiation probe 10 and end portions of the outer covers 181 and 182, the end portions being in the axial direction. The outer cover 183 is configured, for example, as a cap attached in an elastically expanded state. After the outer cover 183 has been removed, the outer cover 183 removed may be cleaned, sterilized, and reused for the irradiation probe 10, or another hygienic outer cover 183 or a brand new outer cover 183 may be attached to the irradiation probe 10. The outer covers 18 thus include the outer cover 183 that is both removable and detachable.

As described above, in a case where the irradiation probe 10 has the plural outer covers 18 that are removable or detachable, for example, only any outer cover 18 that has become unsuitable for use or unusable by becoming unclean or being damaged is able to be removed or replaced, and the running cost is thus able to be reduced as compared to a case where all of the outer covers 18 are removed and replaced.

Furthermore, in a case where the irradiation probe 10 has the plural outer covers 18 at different positions along the axis, only any outer cover 18 that has become unsuitable for use or unusable is able to be removed or replaced among all of the outer covers 18, and more efficient use of the irradiation probe 10 is thus enabled. In particular, the distal end of the irradiation probe 10 tends to become unclean by coming into contact with, for example, a living body in a mode of use where the irradiation probe 10 is used to emit light from the end portion 10b; the outer periphery of the leakage portion 11 and the distal end tend to become unclean by coming into contact with, for example, the living body in a mode of use where the irradiation probe 10 is used to emit light from the outer periphery of the leakage portion 11; and the configuration like this modified example, in which the outer cover 183 covering the distal end including the end portion 10b and the outer cover 182 covering the outer peripheral surface 10c that is the outer periphery of the leakage portion 11 are each removable or detachable, can thus be said to be suitable for these modes of use of the irradiation probe 10.

Furthermore, in a case where the outer cover 181 inside the outer cover 182 is fixed unremovably to the irradiation probe 10 like in this modified example, there is an advantage that protection is able to be achieved by the outer cover 181 even in a state where the outer cover 182 has been temporarily removed for replacement, for example.

### Second Modified Example of Outer Cover that is Removable

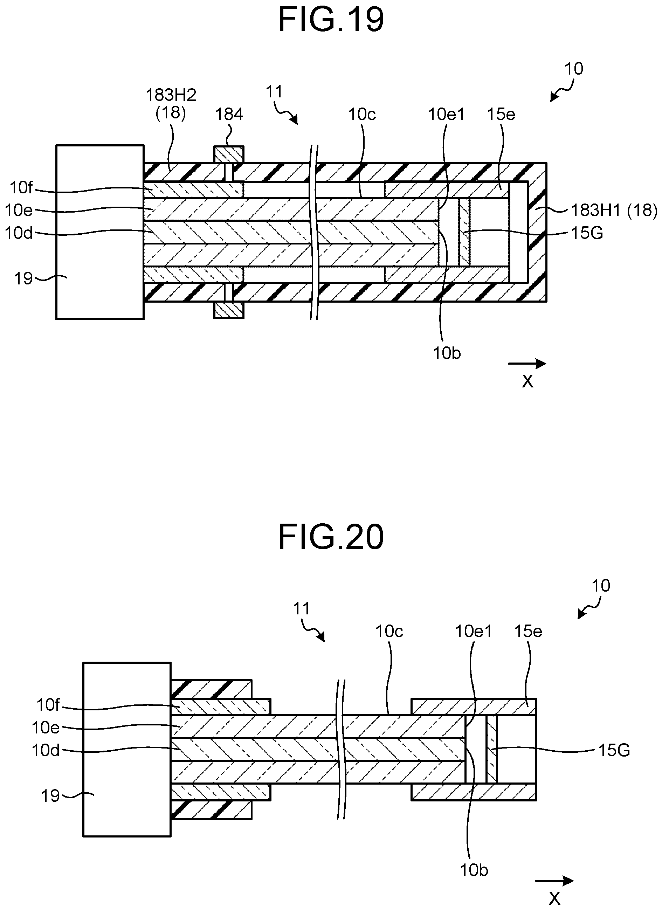

FIG. 19 is a sectional view of an irradiation probe 10 of a modified example of the embodiment. In the example of FIG. 19 also, the irradiation probe 10 has plural outer covers 18 at different positions along its axis. In this modified example, the irradiation probe 10 includes two outer covers 183H1 and 183H2 as the outer covers 18.

The outer cover 183H2 covers a root portion of the body of the irradiation probe 10, the body excluding the outer covers 18, the root portion being near a connector 19, and the outer cover 183H1 covers a portion from an intermediate portion to a distal end portion of the body, the portion excluding the root portion. The outer cover 183H2 has a tubular shape and covers the outer periphery of the root portion, and the outer cover 183H1 has a tubular shape with a bottom and covers the outer periphery of the intermediate portion, the outer periphery of the distal end portion, and an end portion in an axial direction. The outer covers 183H1 and 183H2 are configured to be separable in the axial direction. The outer periphery of a boundary between the outer covers 183H1 and 183H2 is covered with a protection member 184 that is approximately ring-shaped, so that liquid is prevented from coming in from the boundary. The protection member 184 is, for example, a tape, a band, a cover, a ring, or plural hard members that are spreadably or separably connected to each other.

The outer cover 183H2 is fixed to the root portion by, for example, bonding. Because the outer cover 183H2 is connected to the connector 19, the outer cover 183H2 is difficult to be removably or detachably configured. However, the outer cover 183H1 is detachably configured. The outer cover 183H1 may be separably integrated with the irradiation probe 10 by a peelable bonding agent (adhesive) or an attachment tool, such as the protection member 184, or may be elastically fitted with the irradiation probe 10.

FIG. 20 is a sectional view of the irradiation probe 10 illustrated in FIG. 19 in a state where the outer cover 183H1 has been removed. As illustrated in FIG. 20, the protection member 184 has been removed with the outer cover 183H1, from the irradiation probe 10. Thereafter, the outer cover 183H1 removed from the irradiation probe 10 may be cleaned and sterilized to be reused, or another hygienic outer cover 183H1 or a brand new outer cover 183H1 may be attached to the irradiation probe 10. After the outer cover 183H1 has been attached, the protection member 184 is attached. The protection member 184 removed from the irradiation probe 10 may be cleaned and sterilized to be reused, or another hygienic protection member 184 or a brand new protection member 184 may be attached to the irradiation probe 10. This modified example also enables the irradiation probe 10 to be maintained hygienic comparatively easily. Furthermore, this modified example enables provision of a partially removable or detachable portion, additionally to a portion that is inseparable, for the outer covers 18, even in a case where a structure like the connector 19, which is inseparable from the outer cover 18, is included.

### Third Modified Example of Outer Cover that is Removable

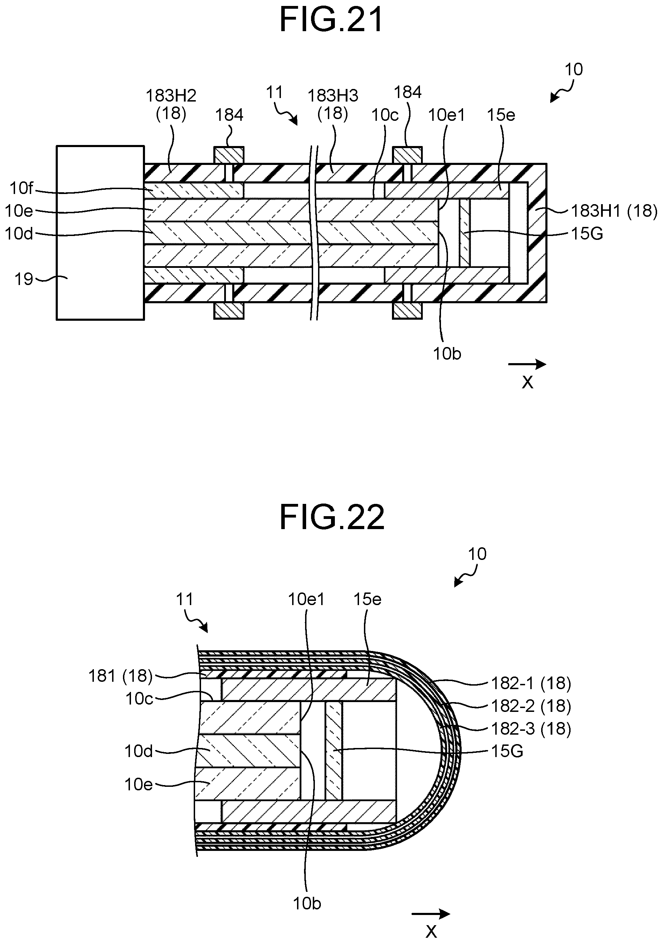

FIG. 21 is a sectional view of an irradiation probe 10 of a modified example of the embodiment. The irradiation probe 10 in FIG. 21 has outer covers 18 similar to those of the irradiation probe 10 in FIG. 19 and FIG. 20. However, in the example of FIG. 21, the outer covers 18 include three outer covers 183H1 to 183H3 at different positions along an axis. Specifically, the outer cover 183H1 covers the outer periphery of a distal end portion and an end portion in an axial direction, the outer cover 183H2 covers the outer periphery of a root portion, and the outer cover 183H3 covers the outer periphery of an intermediate portion. The outer covers 183H1 and 183H3 are detachably configured. This modified example also enables the irradiation probe 10 to be maintained hygienic comparatively easily. The number of the outer covers 18 at the different positions along the axis is not limited to two or three, and may be four or more. Furthermore, the positions of the outer covers 18 along the axis may be changed as appropriate.

### Fourth Modified Example of Outer Cover that is Removable

FIG. 22 is a sectional view of a distal end portion of an irradiation probe 10 of a modified example of the embodiment. The irradiation probe 10 in the example of FIG. 22 has an outer cover 181, similarly to the irradiation probe 10 in the example of FIG. 18. However, the irradiation probe 10 in this modified example has plural (three in this example) outer covers 182-1 to 182-3 that: cover a portion from a distal end portion to an intermediate portion of the body of the irradiation probe 10; are multiply layered over one another; and each have a tubular shape with a bottom. These outer covers 182-1 to 182-3 that have been multiply layered over one another are configured to be attached with, for example, a peelable bonding agent (adhesive) and to be able to be peeled off, that is, removed, one by one from the outside. Therefore, in a case where the outermost outer cover 182-1 has become unclean or has been damaged, a user is able to use the irradiation probe 10 by peeling off the outer cover 182-1, the irradiation probe 10 having the outer cover 182-2 inside the outer cover 182-1, the outer cover 182-2 then being the outer cover exposed outside. Furthermore, in a case where the outer cover 182-2 has become unclean or has been damaged, a user is able to use the irradiation probe 10 by peeling off the outer cover 182-2, the irradiation probe 10 having the outer cover 182-3 inside the outer cover 182-2, the outer cover 182-3 then being the outer cover exposed outside. As described above, outer covers 18 include the outer covers 182-1 to 182-3 that are removable. This modified example also enables the irradiation probe 10 to be maintained hygienic comparatively easily. The number of the outer covers 18 to be layered over one another is not limited to three and may be two, or four or more.

As described above, the irradiation probe system 1 of the embodiment enables irradiation of a region facing the outer peripheral surface 10c of the irradiation probe 10, in addition to a region facing the end portion 10b of the irradiation probe 10, with light. That is, the embodiment enables increase in the irradiation range. Furthermore, the irradiation probe system 1 of the embodiment: enables switchover between a state where only the region facing the end portion 10b is irradiated with light, a state where only the region facing the outer peripheral surface 10c is irradiated with light, and a state where both the region facing the end portion 10b and the region facing the outer peripheral surface 10c are irradiated with light; also enables change in the intensity of irradiation in each irradiation region; and thus has an advantage of enabling flexible change in the irradiation state depending on the situation and achieving appropriate irradiation states of light in various situations.

As described above, the irradiation probe 10 and the irradiation probe system 1 including the irradiation probe 10, which have a larger irradiation range and enable change in the irradiation range and irradiation output, are able to be used in PDT. In a case where they are used in PDT, for example, a beneficial medical system is able to be constructed, the beneficial medical system having various effects, such as enabling faster treatment and enabling more reliable or more appropriate treatment.

Furthermore, the irradiation probe system 1 including the light receiving unit 250 and the detection unit 215 is able to be used in photodynamic diagnosis (PDD). In this case, because it is able to be used in both PDT and PDD, for example, an even more beneficial medical system is able to be constructed, the even more beneficial medical system having various effects, such as being compact and easy to use and facilitating implementation of more appropriate treatment.

The embodiment of the present invention has been described above by way of example, but the embodiment is just an example and is not intended to limit the scope of the invention. The above described embodiment can be implemented in various other modes, and without departing from the gist of the invention, various omissions, substitutions, combinations, and modifications can be made. Furthermore, it may be implemented by modifying, as appropriate, the specifications of the components and shapes (such as, the structures, types, directions, models, sizes, lengths, widths, thicknesses, heights, numbers, arrangements, positions, and materials), for example.

### Industrial Applicability

The present invention is able to be used for irradiation probe systems and irradiation probes.

### Reference Signs List

1 IRRADIATION PROBE SYSTEM
10 IRRADIATION PROBE
10a END PORTION
10a1 END SURFACE (FIRST INPUT END PORTION)
10a2 END SURFACE (SECOND INPUT END PORTION)
10a3 END SURFACE
10b END PORTION (OUTPUT END PORTION)
10b1 INCLINED SURFACE (FIRST OPTICAL ELEMENT, SECOND OPTICAL ELEMENT)
10c OUTER PERIPHERAL SURFACE
10d CORE
10e FIRST CLADDING
10e1 END PORTION
10f SECOND CLADDING (FIRST COVERING LAYER)
11 LEAKAGE PORTION
11a RECESSED PORTION
11b PARTICLE
11c HOLE
12 TRANSMISSION PORTION
13 COVERING LAYER (SECOND COVERING LAYER)
14 SCATTERING LAYER
14a SCATTERING ELEMENT
15A TO 15E OPTICAL ELEMENT (FIRST OPTICAL ELEMENT, SECOND OPTICAL ELEMENT)
15F OPTICAL ELEMENT (THIRD OPTICAL ELEMENT)
15G OPTICAL ELEMENT
15a INCLINED SURFACE (FIRST OPTICAL ELEMENT, SECOND OPTICAL ELEMENT)
15b CONICAL SURFACE (FIRST OPTICAL ELEMENT, SECOND OPTICAL ELEMENT)
15c LENS (FIRST OPTICAL ELEMENT, SECOND OPTICAL ELEMENT)
15d CONVEX CURVED SURFACE (FIRST OPTICAL ELEMENT, SECOND OPTICAL ELEMENT)
15f INNER END SURFACE (REFLECTION PORTION)
15e CYLINDER
16 SOCKET
16a INNER PERIPHERAL SURFACE
17 SCREW MECHANISM (ATTACHMENT AND DETACHMENT MECHANISM)
18, 181, 182, 182-1, 182-2, 182-3, 183, 183H1, 183H2, 183H3 OUTER COVER
19 CONNECTOR
20 DELIVERY OPTICAL FIBER
20a CORE
20b CLADDING
20c OUTER COVER
21 DELIVERY OPTICAL FIBER (FIRST TRANSMISSION OPTICAL FIBER)
22 DELIVERY OPTICAL FIBER (SECOND TRANSMISSION OPTICAL FIBER)
30 COUPLING PORTION
31 TAPERED PORTION
32 STRAIGHT LINED PORTION
33 SWITCHOVER MECHANISM
33a MIRROR
100 OPTICAL OUTPUT DEVICE
110 LIGHT SOURCE UNIT
111 LIGHT SOURCE UNIT
112 LIGHT SOURCE UNIT
150 ATTACHMENT
184 PROTECTION MEMBER
200 CONTROL DEVICE
210 CONTROLLER
211 IRRADIATION CONTROL UNIT
212 INPUT CONTROL UNIT
213 OUTPUT CONTROL UNIT
214 SWITCHOVER CONTROL UNIT
215 DETECTION UNIT
220 INPUT UNIT
230 OUTPUT UNIT
241 MAIN STORAGE UNIT
242 AUXILIARY STORAGE DEVICE
250 LIGHT RECEIVING UNIT
Ax OPTICAL AXIS
La FIRST LIGHT
Lc SECOND LIGHT
X AXIAL DIRECTION

## Claims

1. An irradiation probe system, comprising:
an irradiation probe having a core and first cladding surrounding the core;
at least one light source; and
a coupling portion that couples light output by the at least one light source, to at least one of the core and the first cladding, wherein
the core includes a first input end portion at one end of an axis of the core, and an output end portion that is at the other end of the axis, and
the first cladding includes: a second input end portion at one end of the axis; and a leakage portion that is provided at a position separate from the second input end portion and leaks, radially outward, light transmitted inside the first cladding, from an outer peripheral surface of the first cladding.

2. The irradiation probe system according to claim 1, wherein light transmitted inside the core is output from the output end portion.

3. The irradiation probe system according to claim 1 or 2, comprising, as the at least one light source: a light source that outputs light to be coupled to the core; and a light source that outputs light to be coupled to the first cladding.

4. The irradiation probe system according to any one of claims 1 to 3, wherein the irradiation probe includes a first covering layer between the second input end portion and the leakage portion, the first covering layer surrounding the outer peripheral surface of the first cladding.

5. The irradiation probe system according to claim 4, wherein the first covering layer includes second cladding having a refractive index lower than that of the first cladding.

6. The irradiation probe system according to any one of claims 1 to 5, wherein the irradiation probe includes an outer cover surrounding a surface of the irradiation probe, the surface being radially outermost.

7. The irradiation probe system according to claim 6, wherein the outer cover is made of a resin material.

8. The irradiation probe system according to any one of claims 1 to 5, wherein the irradiation probe includes a section at a position separate, along the axis, from the leakage portion, the section being where the outer peripheral surface of the first cladding is exposed.

9. The irradiation probe system according to any one of claims 1 to 8, wherein the irradiation probe includes a second covering layer that surrounds the core or the first cladding, the second covering layer being on an opposite side of the leakage portion, the opposite side being opposite to a side where the first input end portion is along the axis, and that reduces coupling of light from outside to the core.

10. The irradiation probe system according to claim 9, wherein the second covering layer reduces output, along the axis, of the light transmitted inside the first cladding.

11. The irradiation probe system according to any one of claims 1 to 8, wherein the irradiation probe includes a second covering layer that surrounds the core or the first cladding, the second covering layer being on an opposite side of the leakage portion, the opposite side being opposite to a side where the first input end portion is along the axis, and that reduces output, along the axis, of the light transmitted inside the first cladding.

12. The irradiation probe system according to any one of claims 1 to 11, comprising a first optical element that directs, at the output end portion, light transmitted in the core, radially outward.

13. The irradiation probe system according to claim 12, wherein the first optical element is configured to be detachable.

14. The irradiation probe system according to any one of claims 1 to 13, wherein the leakage portion includes a recessed portion or a protruding portion provided on the outer peripheral surface of the first cladding.

15. The irradiation probe system according to any one of claims 1 to 14, wherein the leakage portion includes particles or holes provided inside the first cladding.

16. The irradiation probe system according to any one of claims 1 to 15, wherein the leakage portion includes a section where the outer peripheral surface of the first cladding changes in shape along the axis.

17. The irradiation probe system according to any one of claims 1 to 16, wherein the leakage portion includes a curved portion of the first cladding.

18. The irradiation probe system according to any one of claims 1 to 17, wherein the leakage portion includes a scattering layer that: is provided radially outside the first cladding; and propagates and scatters, radially outward, the light from the first cladding.

19. The irradiation probe system according to any one of claims 1 to 18, wherein
the first input end portion and the second input end portion are arranged in a radial direction at one end of the axis, and
the coupling portion couples light output from one of the at least one light source to the core and couples light output from another one of the at least one light source to the first cladding.

20. The irradiation probe system according to any one of claims 1 to 19, wherein the coupling portion is optically connected to the core and the first cladding.

21. The irradiation probe system according to any one of claims 1 to 20, wherein the coupling portion includes a fiber bundle that is a bundle of: a first transmission optical fiber that transmits light output by one of the at least one light source and coupled to the core; and a second transmission optical fiber that transmits light output by another one of the at least one light source and coupled to the first cladding.

22. The irradiation probe system according to claim 21, wherein the fiber bundle is a tapered fiber bundle that is tapered toward the irradiation probe.

23. The irradiation probe system according to any one of claims 1 to 22, comprising:
a switchover mechanism that performs selective coupling of light output by the light source to the core or to the first cladding; and
a first control unit that controls the selective coupling by the switchover mechanism.

24. The irradiation probe system according to claim 23, comprising:
an operation input unit for a user, wherein
the first control unit controls the selective coupling by the switchover mechanism according to input of an operation by the user to the operation input unit.

25. The irradiation probe system according to any one of claims 1 to 24, comprising:
plural light sources as the at least one light source, and
a second control unit that performs switchover between light sources to output light, among the plural light sources.

26. The irradiation probe system according to claim 25, wherein the plural light sources include a light source that outputs light to be coupled to the core and a light source that outputs light to be coupled to the first cladding.

27. The irradiation probe system according to claim 25 or 26, wherein the plural light sources include plural light sources that output light to be coupled to the core or plural light sources that output light to be coupled to the first cladding.

28. The irradiation probe system according to any one of claims 25 to 27, comprising:
an operation input unit for a user, wherein
the second control unit performs switchover between light sources to output light, among the plural light sources, according to input of an operation by the user to the operation input unit.

29. The irradiation probe system according to any one of claims 1 to 28, comprising a detection unit that detects light coupled to the output end portion, transmitted inside the core, and output from the first input end portion.

30. The irradiation probe system according to claim 29, wherein the irradiation probe includes a second optical element that couples light coming from outside, to the output end portion.

31. The irradiation probe system according to claim 30, wherein the second optical element is configured to be detachable.

32. The irradiation probe system according to any one of claims 1 to 31, comprising:
a third optical element: having a reflection portion that reflects the second light output from the other end of the axis of the irradiation probe; and configured to be detachable from the irradiation probe, wherein
the irradiation probe system is configured so that the second light reflected by the reflection portion is coupled to the first cladding, at the other end.

33. The irradiation probe system according to any one of claims 1 to 32, wherein the irradiation probe includes an outer cover that is removable or detachable.

34. The irradiation probe system according to claim 33, wherein the irradiation probe includes, as the outer cover, plural outer covers that are removable or detachable.

35. The irradiation probe system according to claim 34, wherein the irradiation probe includes, as the outer cover, plural outer covers that are at different positions along the axis.

36. The irradiation probe system according to claim 35, wherein the irradiation probe includes, as the outer cover, an outer cover covering the output end portion and an outer cover covering an outer periphery of the leakage portion.

37. The irradiation probe system according to any one of claims 33 to 36, wherein the irradiation probe includes a fixed outer cover and an outer cover that covers the fixed outer cover removably or detachably.

38. The irradiation probe system according to any one of claims 33 to 37, wherein the irradiation probe includes, as the outer cover, plural outer covers that have been layered over one another.

39. The irradiation probe system according to claim 38, wherein the plural outer covers that have been layered over one another are configured to be removable one by one from outside.

40. The irradiation probe system according to any one of claims 33 to 39, wherein the outer cover is provided at a portion that comes into contact with a living body upon use for the living body.

41. The irradiation probe system according to any one of claims 33 to 40, wherein the outer cover is made of a biocompatible resin material.

42. An irradiation probe comprising a core and first cladding surrounding the core, wherein
the core includes: a first input end portion at one end of an axis of the core; and an output end portion that is at the other end of the axis, and
the first cladding includes: a second input end portion at one end of the axis; and a leakage portion that is provided at a position separate from the second input end portion and leaks, radially outward, light transmitted inside the first cladding, from an outer peripheral surface of the first cladding.

43. The irradiation probe according to claim 42, wherein light transmitted inside the core is output from the output end portion.

44. The irradiation probe according to claim 42 or 43, comprising a first covering layer between the second input end portion and the leakage portion, the first covering layer surrounding the outer peripheral surface of the first cladding.

45. The irradiation probe according to claim 44, wherein the first covering layer includes second cladding having a refractive index lower than that of the first cladding.

46. The irradiation probe according to any one of claims 42 to 45, comprising an outer cover surrounding a surface of the irradiation probe, the surface being radially outermost.

47. The irradiation probe system according to claim 46, wherein the outer cover is made of a resin material.

48. The irradiation probe according to any one of claims 42 to 47, wherein the leakage portion includes particles or holes provided inside the first cladding.

49. The irradiation probe according to any one of claims 42 to 48, comprising an outer cover that is removable or detachable.
